Europäisches Patentamt

European Patent Office

Office européen des brevets

(11) Veröffentlichungsnummer: **0 254 945 B1**

# EUROPÄISCHE PATENTSCHRIFT

(45) Veröffentlichungstag der Patentschrift: **12.06.91**

(51) Int. Cl.⁵: **G01P 15/08, A61B 5/11**

(21) Anmeldenummer: **87110092.1**

(22) Anmeldetag: **13.07.87**

(54) **Herzschrittmacher mit einem Sensor zum Erfassen der Trägheits- und/oder Rotationsbewegungen eines Gegenstandes oder Lebewesens.**

(30) Priorität: **15.07.86 DE 3623905**

(43) Veröffentlichungstag der Anmeldung:
**03.02.88 Patentblatt 88/05**

(45) Bekanntmachung des Hinweises auf die
Patenterteilung:
**12.06.91 Patentblatt 91/24**

(84) Benannte Vertragsstaaten:
**DE FR GB IT NL SE**

(56) Entgegenhaltungen:
**EP-A- 0 080 348        EP-A- 0 171 848
WO-A-83/00218        DE-A- 2 430 710
DE-A- 3 041 647        FR-A- 1 139 413
FR-A- 2 535 060**

(73) Patentinhaber: **Siemens Elema AB
Röntgenvägen 2
S-171 95 Solna 1(SE)SE**

Patentinhaber: **Siemens Aktiengesellschaft
Wittelsbacherplatz 2
W-8000 München 2(DE)DE FR GB IT NL**

(72) Erfinder: **Lekholm, Anders, Dr.
Gnejsvägen 4
S-161 39 Bromma(SE)**
Erfinder: **Säll, Gösta
Slagstagardsv. 39
S-145 73 Norsborg(SE)**
Erfinder: **Wecke, Liliane, Dipl.-Ing.
Hagalundsg. 46
S-171 50 Solna(SE)**
Erfinder: **Högnelid, Kurt, Dipl.-Ing.
Tulegatan 27 B
S-172 32 Sundbyberg(SE)**

## Beschreibung

Die Erfindung betrifft generell einen implantierbaren Herzschrittmacher mit einem darin angeordneten Sensor zum Erfassen der Bewegungen eines Lebewesens. Für physiologisch gesteuerte Herzschrittmacher besteht der Wunsch, die physische Aktivität des Patienten auf einfache Art zu bestimmen und als Steuerparameter für die Frequenz des Herzschrittmachers zu benutzen. Aus der US-Patentschrift 4 428 378 ist bereits ein derartiger Herzschrittmacher bekannt, der als Sensor beispielsweise ein Mikrofon verwendet. Dadurch werden jedoch neben den gewünschten Aktivitäten auch eine Reihe von Störsignalen miterfaßt wie beispielsweise Atem-und Herzgeräusche oder solche, die von außerhalb des Patienten herstammen können. Die vom Sensor aufgenommenen Störungen hängen dabei auch noch von der Beschaffenheit des Patienten, d.h. seiner Korpulenz oder Muskulosität ab, so daß aufwendige und von Patient zu Patient unterschiedliche Empfindlichkeitsjustierungen und Störunterdrückungen vorgenommen werden müssen.

Außerhalb des Herzschrittmachergebietes sind Sensoren der im Patentanspruch 1 genannten und auch anderer Art bekannt. Beispielsweise ist aus der EP-A0 171 848 ein Bewegungssensor, z.B. zum Überwachen von Lebewesen, bekannt. Der Sensor besteht hier aus einem Hohlkörper (z.B. Messing) mit einem eingeschlossenen Körper (z.B. Stahl). Als Signalumwandler zwischen Bewegungen und elektrischen Signalen dient ein Piezo-Element. Der Hohlkörper/Körper kann aus einem regelmäßigen Polyeder bestehen.

Ein Lageänderungssensor, z.B. zur Diebstahlsicherung, ist durch die DE-A-2 430 710 bekannt. Der Sensor besteht hier aus einer (zylindrischen) Induktionsspule und einem im Spuleninnenraum frei beweglichen Magneten (magnetisierte Stahlkugel/Ellipsoid/Vielflächner). Die Spule ist sogleich Hohlkörperbildner und Umwandler.

WO-A 83/00218 zeigt einen Bewegungssensor z.B. zur Maschinenüberwachung. Der Sensor besteht hier aus einem Hohlkörper, der mit Flüssigkeit von einer ersten bestimmten Lichtdurchlässigkeit und mit Partikeln von einer zweiten bestimmten Lichtdurchlässigkeit gefüllt ist. Zwischen zwei entgegenstehenden Punkten (Sender, Empfänger) an der Hohlkörperwand ist z.B. ein Lichtstrahl angeordnet. Vibriert der Sensor, verteilen sich die Partikeln in der Flüssigkeit anders als im Ruhezustand, und der Absorptionskoeffizient im Lichtweg zeigt entsprechend zwei verschiedene Werte, die in elektrische Signale umgewandelt werden können.

FR-A 1 139 413 beschreibt einen Verzögerungssensor für Fahrzeuge. Bei diesem Sensor rollt eine leitende Kugel über eine als Hohlkörperquadrant ausgebildete Bahn. Die Bahn besteht aus zwei verschiedenen (leitende, resistive) "Schienen", die durch die Kugel verbunden sind. Die "Schienen" sind Teile eines elektrischen Kreises, der seine Resistanz mit der zurückgelegten Bahnlänge der Kugel verändert. Durch die Ausbildung der Bahn zeigt dieser Sensor unterschiedliche Empfindlichkeit in verschiedenen Richtungen.

FR-A-2 535 060 zeigt einen Bewegungssensor für Alarmzwecke. Der Sensor besteht hier aus einer Metallmembran, auf der eine oder mehrere Kugeln rollen und/oder zusammenstoßen. Die Vibrationen werden mittels eines, an die Membran geklebten Piezo-Elementes in elektrische Signale umgewandelt. Die Membran und die Kugeln können in einem Hohlkörper aus Plastik eingeschlossen sein.

Der vorliegenden Erfindung liegt die Aufgabe zugrunde, einen Herzschrittmacher mit einem darin angeordneten, im Aufbau einfachen, möglichst störunabhängigen Sensor anzugeben.

Diese Aufgabe wir durch die Merkmale des Anspruchs 1 gelöst.

Der im Herzschrittmacher angeordnete Sensor besteht demnach aus einem Hohlkörper, in dem mindestens ein vorteilhafterweise regelmäßiger Körper frei bewegbar ist. So kann beispielsweise der Körper aus einem regelmäßigen Polyeder aus einem harten Material bestehen. Alternativ dazu kann der Körper sphärisch ausgebildet sein, während die Innenseite des Hohlkörpers facettiert ist.

Zur Erläuterung der Funktionsweise dieses Sensors wird beispielhaft angenommen, daß der Hohlkörper innen sphärisch aufgebaut ist und als Körper ein Ikosaeder oder Dodekaeder verwendet wird. Wenn sich der Gegenstand oder das Lebewesen, dessen Trägheits- oder Rotationsbewegungen bestimmt werden sollen, in Ruhe befindet, liegt der Körper auf dem Boden des Hohlkörpers auf einer seiner Flächen. Wenn der Hohlkörper einer Beschleunigung oder einer langsamen Rotation ausgesetzt wird, wird sich der Körper so lange zusammen mit dem Hohlkörper bewegen, bis sein Schwerpunkt die Kante der Oberfläche erreicht, auf der er ruht. Eine weitere Rotation wird dazu führen, daß der Körper auf eine angrenzende Phase rollt, wo sich ein neues Gleichgewicht einstellt. Bei diesem Überrollen auf die neue Fläche wird ein mechanischer Stoß erzeugt. Dieser Stoß kann als ein klickender Laut gehört werden und durch den vorhandenen Übertrager erfaßt werden. Der Übertrager kann dazu typisch ein Mikrofon sein. Alternativ ist es ebenso denkbar, daß der Körper aus einem permanentmagnetischen Material besteht und als Übertrager eine oder mehrere Spulen vorgesehen sind, in denen ein Strom erzeugt wird, wenn sich der Körper im inneren des Hohlkörpers bewegt.

Die Bewegungen des Körpers sind aufgrund seiner Form und der Form des Hohlkörpers "quantisiert". Wenn sich der Körper relativ zum Hohlkörper bewegt, produziert er eine Kombination von Signalen, die aus einem Geräusch bestehen, dass durch die gleitende Bewegung des Körpers hervorgerufen wird und aus klickenden Geräuschen, die durch die rollende Bewegung des Körpers hervorgerufen werden. Die vom Übertrager entsprechend aufgenommenen Signale können beispielsweise nach einer Verstärkung mittels einer Schwellwertschaltung und eines Impulsformers behandelt werden, so dass einheitliche Impulse gleicher Amplitude und Breite erzeugt werden, wobei jeder Impuls der Verschiebung des Körpers von einer Fläche zur anderen entspricht. Das so erzeugte Signal, d.h. die Frequenz dieser Impulse kann dann als Mass für die Intensität der Beschleunigung oder Rotationsbewegung dienen, denen der Sensor ausgesetzt ist.

Wenn die Innenwand des Hohlkörpers elastisch ist, erregen die Stösse des Körpers gedämpfte mechanische Schwingungen. Die Frequenz dieser Schwingungen wird von den mechanischen Eigenschaften, d.h. der Elastizität der Innenwand des Hohlkörpers bestimmt. Eine Detektion dieser Schwingungen mit einem schmalbandigen Verstärker führt zu einer hohen Unterdrückung von Störsignalen.

Vorteilhafterweise kann die Empfindlichkeit des Sensors einfach durch Variation des Aufbaus geändert werden, ohne dass dabei die relative Korrektheit der Messung verloren geht.

In vorteilhafter Weiterbildung der Erfindung ist vorgesehen, dass die Empfindlichkeit des im Herzschrittmacher angeordneten Sensors in verschiedenen Richtungen und/oder Lagen unterschiedlich ist. Das kann beispielsweise durch Änderung der Form des Hohlkörpers von sphärischer (isotroper Empfindlichkeit) zu ellipsoider erreicht werden. Ebenso einfach ist es, gewisse Teile des Hohlkörpers aus einem weicheren Material herzustellen als andere. Eine weitere Möglichkeit besteht darin, einen sphärischen Körper zu verwenden und bestimmte innere Oberflächen des Hohlkörpers glatt und andere facettiert zu gestalten. Die hervorgerufenen Signalamplituden können dadurch so unterschiedlich ausfallen, dass die beschriebene Signalverarbeitungselektronik die Bewegung des Körpers an bestimmten Stellen des Hohlkörpers oder in bestimmten Richtungen unterdrückt. Das bedeutet aber, dass der Sensor auf diese einfache Art und Weise unempfindlich für Bewegungen in bestimmten Positionen oder Richtungen gemacht werden kann.

Weiterhin kann die Empfindlichkeit des Sensors dadurch geändert werden, dass die relative Grösse des Körpers im Verhältnis zur Grösse des Hohlkörpers geändert wird oder durch Änderung der Form eines dieser Körper. Beispielhaft sei hier nur angeführt, dass ein Ikosaeder einfacher rollen wird als ein Würfel. Höchste Empfindlichkeit wird als Grenzfall mit einer kleinen Kugel in einem grossen sphärischen Hohlkörper erreicht. Da hier die mechanischen Vibrationen extrem klein oder bei idealen Bedingungen sogar Null sein werden, wird für diese Ausgestaltung ein magnetischer Übertrager und ein permanentmagnetischer Körper erforderlich sein.

Weiterhin können die Bewegungen des Körpers in dem Hohlkörper dadurch gezielt beeinflusst werden, dass der Hohlraum mit einer Flüssigkeit wählbarer Viskosität und/oder mit mehreren Teilchen ausgefüllt wird, die zusätzliche detektierbare Druckwellen oder Signale durch ihre kollektive Bewegung gegen die Innenwand des Hohlkörpers oder gegeneinander produzieren.

Bei dem erfindungsgemässen implantierbaren Herzschrittmacher mit einem Impulsgenerator zur Erzeugung frequenzvariabler Stimulierungsimpulse wird mit dem Sensor die physische Aktivität eines Patienten erfasst und das Ausgangssignal des Sensors dient zur Steuerung der Frequenz des Impulsgenerators. Die Verwendung eines derartigen Sensors zur Frequenzsteuerung eines implantierbaren Herzschrittmachers hat gegenüber bekannten Einrichtungen mehrere Vorteile. So bildet der Sensor ein in sich völlig geschlossenes System innerhalb des Herzschrittmachers, dass keine zusätzlichen externen Detektoren oder Leitungsverbindungen ausserhalb des Herzschrittmachers benötigt. Weiterhin kann der Sensor absolut kalibriert werden für Beschleunigungen oder Rotationsbewegungen in einem Bereich, der den normalen Aktivitäten eines Patienten entspricht. Der Sensor muss daher nicht individuell für jeden Patienten angepasst werden. Die einzige möglicherweise notwendige Anpassung bestünde dann in der Justierung der Übertragungsfunktion zu einem Frequenzkontrollsignal für den Impulsgenerator derart, dass eine bestimmte Aktivität in einer optimalen Stimulierungsfrequenz für jeden Patienten resultiert.

Anhand von 18 Figuren werden im folgenden Ausführungsbeispiele der Erfindung näher beschrieben und erläutert. Dabei zeigen

FIG 1    eine erste Sensorausgestaltung mit Übertrager und anschliessender Grundschaltung zur Signalaufbereitung,

FIG 2    räumlich passend zu FIG 1 die verschiedenen Signalformen,

FIG 3    ein Anwendungsbeispiel des erfindungsgemässen frequenzgesteuerten Herzschrittmachers mit einem darin angeordneten Sensor und die

FIG 4-13    weitere mögliche Ausführungsformen des Sensors.

FIG 14-17    eine bevorzugte Sensorausgestaltung nach dem Induktionsprinzip
in einem frequenzgesteuerten
Herzschrittmacher,

FIG 18       die Sensorelektronik mit Signalformen passend zu dem in den

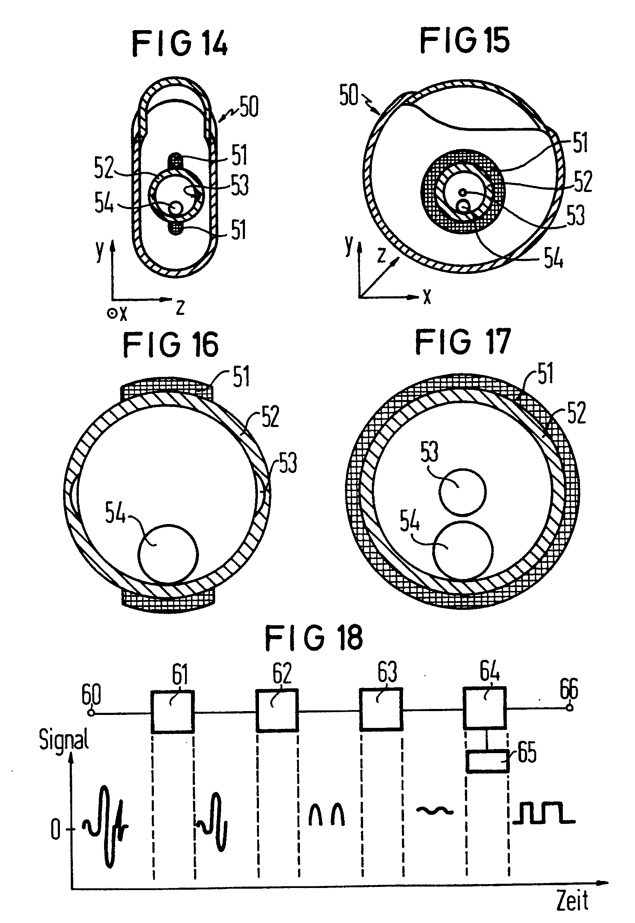

Figuren 14 bis 17 dargestellten
Sensor.

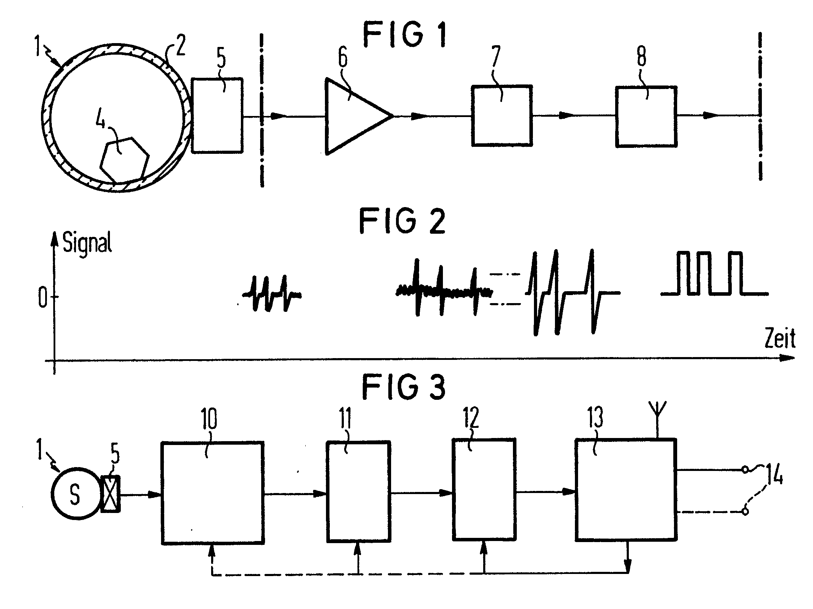

In FIG 1 ist als Sensor 1 eine Hohlkugel 2, beispielsweise aus Glas vorgesehen, in der sich ein regelmässiges Polyeder 4 - beispielsweise aus geschliffenem Stein - befindet. An der Aussenwand der Hohlkugel 2 sitzt ein Mikrofon 5, dass die durch die Relativbewegung zwischen Hohlkugel und Körper hervorgerufenen Geräusche aufnimmt. Das elektrische Ausgangssignal des Mikrofons gelangt über einen Verstärker 6 und eine Schwellwertstufe 7 auf einen Impulsformer 8.

In der entsprechenden Figur 2 sind von links nach rechts über der Zeit die jeweiligen Signale am Ausgang des Mikrofones 5, des Verstärkers 6, der Schwellwertstufe 7 und schliesslich des Impulsformers 8 dargestellt. Die Zeitbasis gilt dabei für jedes einzelne Signal, nicht jedoch für die vier Signale untereinander. Wie man der FIG 2 entnehmen kann, liegen am Ausgang des Impulsformers Sensorimpulse einheitlicher Amplitude und Impulsbreite vor. Lediglich die "Klick"-Laute beim Kippen des Körpers 4 von einer Polyederfläche zur benachbarten werden am Ausgang angezeigt.

FIG 3 zeigt die Verwendung des Sensors 1 mit Mikrofon 5 und Verstärkerstufe, Schwellwertstufe und Impulsformer, die in dieser Figur in einem Block 10 zusammengefasst sind. Das Ausgangssignal des Blockes 10 ist auf einen Impulsraten/Spannungs-Umformer 11 geschaltet und von dort auf eine Schaltstufe 12, die dieses Aktivitätssignal (variierende Spannung) nach einem Algorithmus in ein Kontrollsignal umwandelt, das den Impulsgenerator 13 des Herzschrittmachers ansteuert. Der Algorithmus der Schaltstufe 12 kann dabei linear oder nichtlinear sein. Sämtliche Teile dieser Schaltung können programmierbar sein, so dass das Kontrollsignal individuell für jeden Patienten entsprechend seinen physiologischen Bedingungen angepasst werden kann. Die Ausgangssignale des Impulsgenerators 13 werden über eine oder mehrere Leitung(en) 14 an das Herz abgegeben.

Das Kontrollsignal für den Herzschrittmacher kann auch mit Hilfe einer digitalen Signalverarbeitung anstatt der beschriebenen analogen erzeugt werden. Die digitale Verarbeitung kann einen Mikroprozessor einschliessen. Die Programmierung kann über eine Telemetrie-Verbindung zwischen Herzschrittmacher und einem externen Programmiergerät erfolgen.

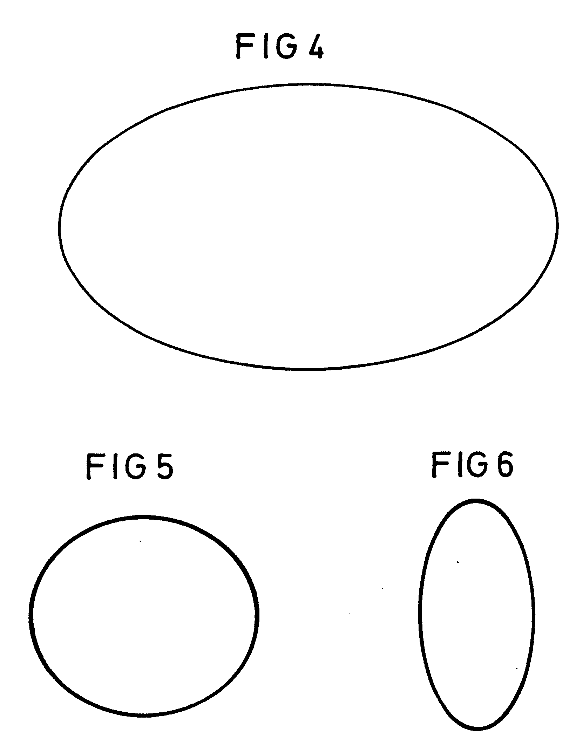

Die FIG 4 bis 6 zeigen einen ellipsoidisch ausgebildeten Hohlkörper, FIG 4 dabei in räumlicher Darstellung, die FIG 5 und 6 als schematische Schnitte in zwei senkrecht zueinander stehenden Ebenen. Denkt man sich als Körper in diesem Hohlkörper beispielsweise ein Polyeder, so wird dieses bei gleicher Körperaktivität unterschiedlich leicht von einer Phase zur anderen überrollen, je stärker die Krümmung der Elipsoide ist.

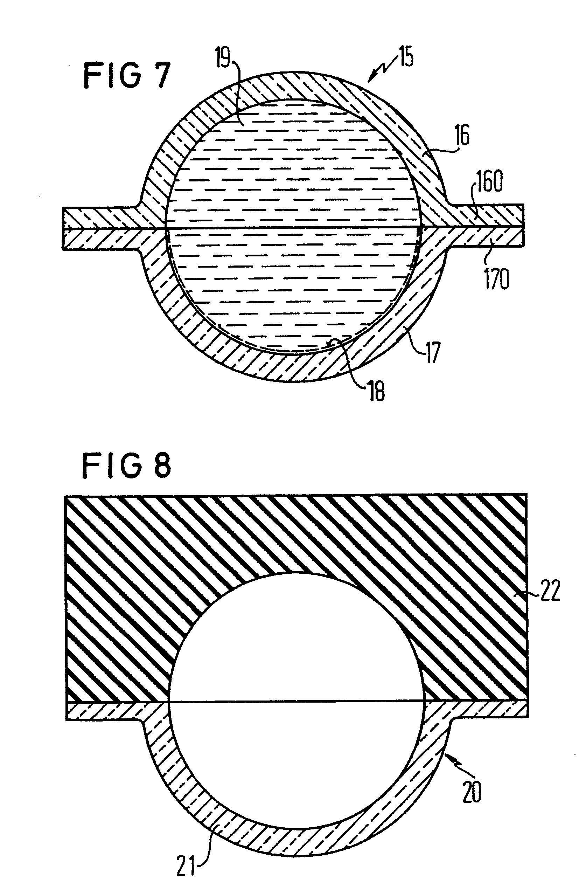

Anhand der FIG 7 ist dargestellt, dass die unterschiedliche Empfindlichkeit des Sensors in verschiedenen Richtungen auch dadurch erzielt werden kann, dass ein sphärischer Hohlkörper 15 aus zwei halbkugelförmigen Schalen 16 bzw. 17 zusammengesetzt ist, wobei beide Schalen beispielsweise aus Glas bestehen können und die Schale 16 eine glatte und die Schale 17 eine strukturierte Innenoberfläche aufweist, wie durch die gestrichelte Linie 18 angedeutet ist. Die Schalen 16 bzw. 17 sind je mit einem Kragen 160 bzw. 170 versehen, wodurch ein dichtes Zusammenfügen dieser beiden Schalen vereinfacht wird.

Als Körper kann wiederum ein Polyeder verwendet werden, dass hier nicht explizit dargestellt ist. Weiterhin ist gemäss FIG 7 der Hohlkörper mit einer Flüssigkeit 19 gefüllt, die die Relativbewegungen des Polyeders in dem Hohlkörper 15 dämpft.

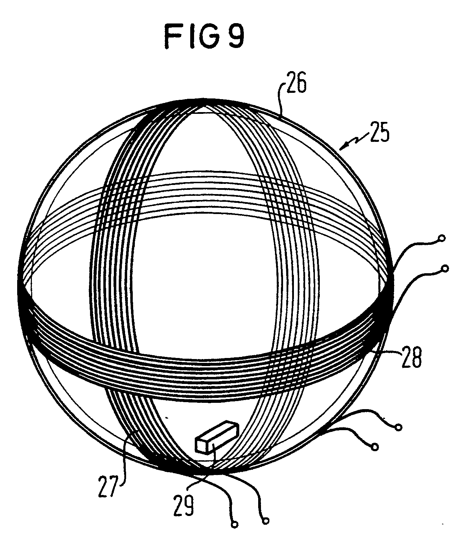

FIG 8 zeigt im Schnitt eine weitere mögliche Ausgestaltung für eine gerichtete Empfindlichkeit des Sensors. Der Sensor 20 besteht ebenfalls aus zwei Halbschalen 21 bzw. 22, wobei die Halbschale 21 wiederum aus Glas, die andere Halbschale 22 jedoch aus einem relativ weichen Gummi besteht.

In den FIG 4 bis 8 ist jeweils nur der Hohlkörper des Sensors dargestellt worden, nicht aber der Übertrager zum Erfassen der Relativbewegung des Körpers im Hohlkörper. Dieser kann beispielsweise wie in der FIG 1 aus einem Mikrofon bestehen, das am oder in der Nähe des Hohlkörpers angeordnet ist.

FIG 9 zeigt ein Ausführungsbeispiel mit einem anderen Übertrager. Der Einfachheit halber ist wieder ein sphärischer Sensor 25 gewählt, auf dessen Umfang drei orthogonal angeordnete Spulen 26, 27 und 28 angeordnet sind. Als Körper dient ein magnetischer Dipol 29. Bei einer Relativbewegung des Magneten 29 wird in den Spulen eine Spannung induziert.

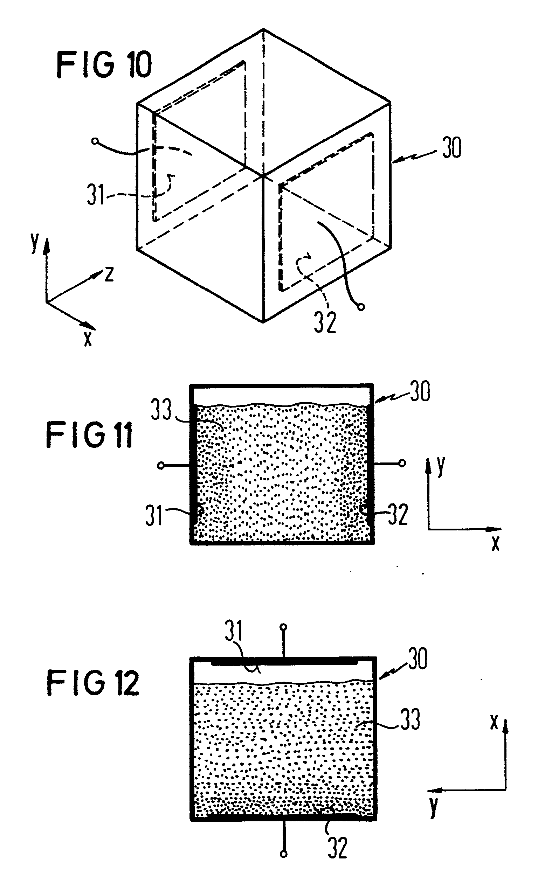

Die FIG 10 bis 12 zeigen eine weitere Ausführungsform des im erfindungsgemässen Herzschrittmacher angeordneten Sensors. FIG 10 zeigt dazu in einer räumlichen Darstellung einen Sensor 30, der aus einem hohlen Kubus besteht, dessen in der yz-Ebene liegenden Innenflächen mit je einer Elektrode 31 bzw. 32 versehen sind. Das Innere dieses Kubus ist zumindest teilweise mit elektrisch leitenden Teilchen, insbesondere Kohlepartikel, lose gefüllt. In der räumlichen Darstellung der FIG 10 sind die Teilchen nicht eingezeichnet. Zur Verdeutlichung der Orientierung und zur Erklärung der folgenden FIG 11 und 12 ist neben dem Kubus noch ein Koordinatensystem dargestellt.

FIG 11 zeigt einen Schnitt durch diesen Kubus entlang einer xy-Ebene, wobei die y-Achse vertikal liegt. Die Elektroden dieses Kubus sind ähnlich einem Kohlemikrofon an eine - hier nicht dargestellte - Spannungsquelle angeschlossen. Wird auf diesen Sensor eine Kraft ausgeübt, indem sich der Patient bewegt, so orientieren sich die Kohlepartikel 33 um, was zu einer Widerstandsänderung zwischen den Elektroden 31 und 32 führt.

In FIG 12 ist der gleiche Schnitt wie in FIG 11 dargestellt mit dem Unterschied, dass diesmal die x-Achse vertikal ausgerichtet ist. Zwischen den Kohlepartikeln 33 und der oberen Elektrode 31 wird sich gem. der FIG 12 eine von Kohlepartikeln 33 freie und damit elektrisch isolierende Schicht bilden, so dass der Stromkreis praktisch in dieser Lage des Sensors unterbrochen ist. Der Sensor kann damit so im Herzschrittmacher angeordnet werden, dass im liegenden Zustand des Patienten kleinere Krafteinwirkungen nicht mehr dedektiert werden.

Der in den FIG 10 bis 12 dargestellte Sensor ist damit in seiner Empfindlichkeit richtungsabhängig. Im Gegensatz zu normalen Kohlemikrofonen kommt es hier nicht darauf an, dass durch Krafteinwirkung - beim Kohlemikrofon die Schallwellen - eine Membran deformiert und damit die Grösse des mit Kohlepartikeln gefüllten Hohlraumes variiert wird. Hier kommt es lediglich darauf an, dass durch die Krafteinwirkung die Kohlepartikel in Bewegung geraten und sich umorientieren.

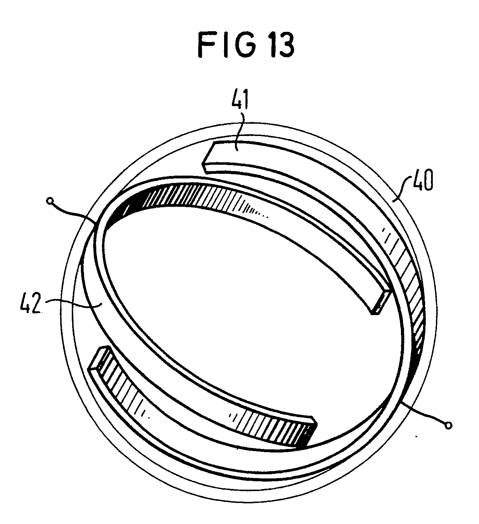

In der FIG 13 ist ein entsprechender Sensor wie in den FIG 10 - 12, aber mit einer isotropen Empfindlichkeit dargestellt. Der Hohlkörper 40 ist dabei wieder sphärisch ausgebildet und auf der Innenseite mit zwei etwa C-bogenförmigen Elektroden 41 bzw. 42 versehen. Der Hohlkörper 40 ist wiederum mit leitenden, hier nicht dargestellten Teilchen lose gefüllt.

FIG 14 bis 17 zeigen ein weiteres Beispiel der Anordnung eines induktiven Sensors in einem frequenzgesteuerten Herzschrittmacher, FIG 14 dabei in schematischer Seitenansicht den Herzschrittmacher mit eingebautem Sensor und FIG 15 die entsprechende Frontalansicht. Die X-Y-Ebene in FIG 14 und 15 entspricht der Ebene, in der der Herzschrittmacher 50 in einem Patienten implantiert ist. Die Figur 16 zeigt passend zu Figur 14 - einen schematischen Schnitt durch den Sensor und Figur 17 zeigt - passend zu Figur 15 - einen schematischen Frontalschnitt durch den Sensor.

Auf dem sphärischen Hohlkörper 52 ist eine ringförmige, aus isoliertem Silber/Kupferdraht bestehende Spule 51 angeordnet. Die Wand des Hohlkörpers 52 besitzt in Z-Richtung zwei Kavitäten 53. Ein magnetischer Dipol hat die Form einer Kugel 54.

Der Sensor ist symmetrisch in der X-Y-Ebene und seine Funktion wird deshalb von einer möglichen Rotation des Herzschrittmachers 50 im Körper nicht beeinflusst. Die zwei Kaviteten 53 sind Ruhepositionen für die Kugel 54. Befindet sich also der Patient in Liegeposition, bewirken die Kaviteten 53, dass ein Schwellwert für die Lageänderung der Kugel 54 überschritten werden muss, bevor der Sensor Frequenzerhöhungen im Herzschrittmacher hervorruft.

Der magnetische Dipol besteht aus einer magnetisierten Stahlkugel 54 oder einem gesinterten, kugelförmigen Pulvermagnet. Ebensogut kann ein nichtsphärischer, in einer Sphäre eingegossener Dipol verwendet werden. Die Kugel oder Sphäre kann auch mit einer äusseren Schicht (z.B. Nickel) versehen werden, die z.B. die Reibung zwischen Hohlkörperwand und Kugel/Sphäre langzeitstabil hält. Im Falle eines Sintermaterials verhindert diese Schicht auch einen möglichen Abrieb.

Der Hohlkörper kann z.B. aus Glas, Keramik, Plexiglas, Thermoplast, härtbarem Kunststoff, Metall, Gummi (z.B. Silikon-Gummi) usw. bestehen. Als Alternative zu einer Sphäre ist eine in Z-Richtung mit kegelförmigen Abschlussdeckeln versehene(r), abgestumpfte Sphäre oder Zylinder vorgesehen.

Aus der Figur 18 sind die verschiedenen Stufen der Signalelektronik und die dazugehörigen Signalformen ersichtlich. Der Eingang ist mit 60 bezeichnet. Das dort erscheinende Signal hat eine Resonanzfrequenz von etwa 10 - 15 Hz. Das Sensorsignal wird zunächst einem Bandpassfilter 61 (5-25 Hz) zugeführt, wonach eine nichtlineare Verstärkungsstufe 62 folgt. Die nächste Stufe ist ein Mittelwertbildner 63 (Kondensator), dessen Ausgang an einem Eingang eines Komparators 64 anliegt. Am anderen Eingang des Komparators ist ein Sägezahnspannungsgenerator 65 angeschlossen. Die Sägezahnspannung sorgt dafür, dass die Spannungsvariation des Mittelwertbildners 63 in eine entsprechende Pulsbreitenvariation umgewandelt wird. Das pulsbreitmodulierte Signal am Ausgang 66 wird dann zur Steuerung der Herzschrittmacherfrequenz herangezogen.

Bezugszeichenliste

| | |
|---|---|
| 1, 20, 30 | Sensor |
| 2 | Hohlkugel (Hohlkörper) |
| 4 | Polyeder (Körper) |
| 5 | Mikrofon (Übertrager) |
| 6 | Verstärker |
| 7 | Schwellwertstufe |
| 8 | Impulsformer |

| 10 | Block |
| 11 | Impulsraten/Spannungs-Umformer |
| 12 | Schaltstufe |
| 13 | Impulsgenerator |
| 14 | Leitungen |
| 15 | sphärischer Hohlkörper (Hohlraum) |
| 16, 17 | Schalen |
| 18 | gestrichelte Linie |
| 19 | Flüssigkeit (fliessendes Medium) |
| 21, 22 | Halbschalen (untersch. Bereiche) |
| 25 | sphärischer Sensor (Hohlkörper) |
| 26, 27, 28, 51 | Spulen |
| 29 | magnetischer Dipol (Körper) |
| 31, 32, 41, 42 | Elektrode (Teile) |
| 33 | Kohlepartikel (Teile) |
| 40, 52 | Hohlkörper |
| 50 | Herzschrittmacher |
| 53 | Kaviteten |
| 54 | Kugel (Körper) |
| 60 | Eingang |
| 61 | Bandpassfilter |
| 62 | Verstärkungsstufe |
| 63 | Mittelwertbildner (Kondensator) |
| 64 | Komparator |
| 65 | Sägezahnspannungsgenerator |
| 66 | Ausgang |
| 160, 170 | Kragen |

## Ansprüche

1. Implantierbarer Herzschrittmacher (50) mit einem Impulsgenerator (13) zur Erzeugung frequenzvariabler Stimulierungsimpulse in Abhängigkeit von der mit einem im Herzschrittmacher (50) angeordneten Sensor (1) gemessenen physischen Aktivität eines Patienten, **dadurch gekennzeichnet,** daß der Sensor (1) aus einem Hohlkörper (2) besteht, in dem mindestens ein Körper (4) frei bewegbar eingeschlossen ist, und daß am Hohlkörper ein Übertrager angeordnet ist, der Relativbewegungen zwischen Hohlkörper (2) und Körper (4) erfaßt und in ein elektrisches Signal umwandelt, das wenigstens teilweise der zu erfassenden physischen Aktivität proportional ist und die Frequenz des Impulsgenerators (13) steuert.

2. Implantierbarer Herzschrittmacher nach Anspruch 1, **dadurch gekennzeichnet,** daß der Körper (29, 54) einen magnetischen Dipol einschließt und als Übertrager eine oder mehrere Spulen (26-28, 51) vorgesehen sind, in denen bei einer Relativbewegung zwischen Körper (29, 54) und Hohlkörper (25, 52) ein Strom induziert wird.

3. Implantierbarer Herzschrittmacher nach einem der Ansprüche 1 bis 2, **dadurch gekennzeichnet,** daß der Körper (29, 54) und/oder Hohlkörper sphärisch ausgebildet ist (sind).

4. Implantierbarer Herzschrittmacher nach einem der Ansprüche I bis 3, **dadurch gekennzeichnet,** daß die Innenwand des Hohlkörpers an mindestens einer Stelle eine Vertiefung (53) aufweist.

5. Implantierbarer Herzschrittmacher nach einem der Ansprüche 1 bis 2, **dadurch gekennzeichnet,** daß der Körper sphärisch und der Hohlkörper ellipsoidisch ausgebildet ist.

6. Implantierbarer Herzschrittmacher nach einem der Ansprüche 1 und 3 bis 5, **dadurch gekennzeichnet,** daß die Innenwand des Hohlkörpers (15, 52) - zumindest teilweise - strukturiert ist.

7. Implantierbarer Herzschrittmacher nach einem der Ansprüche 1 und 3 bis 6, **dadurch gekennzeichnet,** daß die Struktur Facetten aufweist.

8. Implantierbarer Herzschrittmacher nach einem der Ansprüche 1 bis 7, **dadurch gekennzeichnet,** daß die Innenwand des Hohlraumes in unterschiedlichen Bereichen (21, 22) aus Materialien verschiedener Härte gebildet ist.

9. Implantierbarer Herzschrittmacher nach einem der Ansprüche 1 bis 8, **dadurch gekennzeichnet,** daß der Hohlraum (15) mit einem fließenden Medium (19) bestimmter Dichte und Viskosität gefüllt ist.

10. Implantierbarer Herzschrittmacher nach einem der Ansprüche 1 bis 9, **dadurch gekennzeichnet,** daß der Körper aus einem festen Material besteht.

11. Implantierbarer Herzschrittmacher nach Anspruch 1 bis 9, **dadurch gekennzeichnet,** daß der Körper (29, 54) aus mehreren Materialien besteht.

12. Implantierbarer Herzschrittmacher nach Anspruch 10, **dadurch gekennzeichnet,** daß das Material permanentmagnetisch ist.

13. Implantierbarer Herzschrittmacher nach einem der Ansprüche 1 bis 12, **dadurch gekennzeichnet**, daß der Körper (4, 54) eine Oberflächenstruktur aufweist.

14. Implantierbarer Herzschrittmacher nach einem der Ansprüche 1 bis 12, **dadurch gekennzeichnet**, daß der Körper (54) eine glatte Oberfläche aufweist.

15. Implantierbarer Herzschrittmacher nach einem der Ansprüche 13 bis 14, **dadurch gekennzeichnet**, daß die Oberfläche des Körpers aus einem äußeren Schichtmaterial gebildet ist.

16. Implantierbarer Herzschrittmacher nach einem der Ansprüche 1, 3, 6, bis 11, 13 bis 15, **dadurch gekennzeichnet**, daß der Körper (4) und/ oder Hohlkörper aus einem regelmäßigen Polyeder besteht (bestehen).

17. Implantierbarer Herzschrittmacher nach Anspruch 1, **dadurch gekennzeichnet**, daß der Hohlkörper mit mehreren Körpern in Form von Teilchen gefüllt ist.

18. Implantierbarer Herzschrittmacher nach Anspruch 17, **dadurch gekennzeichnet**, daß die Teilchen (33) leitend sind und daß Teile (31, 32) der Innenwand des Hohlkörpers leitend ausgebildet und an einer Spannungsquelle angeschlossen sind.

19. Implantierbarer Herzschrittmacher nach Anspruch 1, **dadurch gekennzeichnet**, daß als Übertrager ein Mikrofon (5) vorgesehen ist.

20. Implantierbarer Herzschrittmacher nach Anspruch 1, **dadurch gekennzeichnet**, daß das elektrische Signal die Frequenz des Impulsgenerators nach einer Verstärkung und/oder Signalaufbereitung steuert.

**Claims**

1. Implantable pacemaker (50) having a pulse generator (13) for generating frequency-variable stimulation pulses as a function of the physical activity of a patient measured using a sensor (1) arranged in the pacemaker (50), characterised in that the sensor (1) consists of a hollow body (2) in which at least one body (4) is enclosed in a freely movable manner, and in that arranged on the hollow body there is a transformer which detects relative movements between hollow body (2) and body (4) and converts them into an electrical signal which is at least partially proportional to the physical activity to be detected and controls the frequency of the pulse generator (13).

2. Implantable pacemaker according to Claim 1, characterised in that the body (29, 54) includes a magnetic dipole and one or more coils (26-28, 51) are provided as transformers, in which coils a current is induced in the event of a relative movement between body (29, 54) and hollow body (25, 52).

3. Implantable pacemaker according to one of Claims 1 to 2, characterised in that the body (29, 54) and/or hollow body is (are) of spherical construction.

4. Implantable pacemaker according to one of Claims 1 to 3, characterised in that the inner wall of the hollow body has a depression (53) at at least one point.

5. Implantable pacemaker according to one of Claims 1 to 2, characterised in that the body is of spherical construction and the hollow body of ellipsoidal construction.

6. Implantable pacemaker according to one of Claims 1 and 3 to 5, characterised in that the inner wall of the hollow body (15, 52) is, at least partially, structured.

7. Implantable pacemaker according to one of Claims 1 and 3 to 6, characterised in that the structure has facets.

8. Implantable pacemaker according to one of Claims 1 to 7, characterised in that the inner wall of the hollow space is constructed in different areas (21, 22) of materials of different hardness.

9. Implantable pacemaker according to one of Claims 1 to 8, characterised in that the hollow space (15) is filled with a fluid medium (19) of a specific density and viscosity.

10. Implantable pacemaker according to one of Claims 1 to 9, characterised in that the body consists of a solid material.

11. Implantable pacemaker according to Claim 1 to 9, characterised in that the body (29, 54) consists of a plurality of materials.

12. Implantable pacemaker according to Claim 10, characterised in that the material is permanently magnetic.

13. Implantable pacemaker according to one of Claims 1 to 12, characterised in that the body (4, 54) has a surface structure.

14. Implantable pacemaker according to one of Claims 1 to 12, characterised in that the body (54) has a smooth surface.

15. Implantable pacemaker according to one of Claims 13 to 14, characterised in that the surface of the body is formed from an external layer material.

16. Implantable pacemaker according to one of Claims 1, 3, 6 to 11, 13 to 15, characterised in that the body (4) and/or hollow body consist(s) of a regular polyhedron.

17. Implantable pacemaker according to Claim 1, characterised in that the hollow body is filled with a plurality of bodies in the form of particles.

18. Implantable pacemaker according to Claim 17, characterised in that the particles (33) are conductive, and in that parts (31, 32) of the inner wall of the hollow body are of conductive construction and are connected to a voltage source.

19. Implantable pacemaker according to Claim 1, characterised in that a microphone (5) is provided as transformer.

20. Implantable pacemaker according to Claim 1, characterised in that the electrical signal controls the frequency of the pulse generator after amplification and/or signal processing.

**Revendications**

1. Stimulateur cardiaque implantable (5) possédant un générateur d'impulsions (13) servant à produire des impulsions de stimulation à fréquence variable en fonction de l'activité physique d'un patient, mesurée au moyen d'un capteur (1) disposé dans un stimulateur cardiaque (5), caractérisé par le fait que le capteur (1) est constitué par un corps creux (2), dans lequel au moins un corps (4) est introduit de manière à être librement mobile, et que sur le corps creux est monté un transducteur qui détecte des déplacements relatifs entre le corps creux (2) et le corps (4) et les convertit en un signal électrique qui est au moins partiellement proportionnel à l'activité physique devant être détectée, et commande la fréquence du générateur d'impulsions (13).

2. Stimulateur cardiaque implantable suivant la revendication 1, caractérisé par le fait que le corps (29,54) comporte un dipôle magnétique et qu'il est prévu, comme transducteur, une ou plusieurs bobines (26-28,51), dans lesquelles un courant est induit lors d'un déplacement relatif entre le corps (29,54) et le corps creux (25,52).

3. Stimulateur cardiaque implantable suivant l'une des revendications 1 et 2, caractérisé par le fait que le corps (29,54) et/ou le corps creux est (sont) sphérique(s).

4. Stimulateur cardiaque implantable suivant l'une des revendications 1 à 3, caractérisé par le fait que la paroi intérieure du corps creux possède un renfoncement (53), en au moins un emplacement.

5. Stimulateur cardiaque implantable suivant l'une des revendications 1 et 2, caractérisé par le fait que le corps est sphérique et le corps creux possède la forme d'un ellipsoïde.

6. Stimulateur cardiaque implantable suivant l'une des revendications 1 et 3 a 5, caractérisé par le fait que la paroi intérieure du corps creux (15,52) est -au moins partiellement-structurée.

7. Stimulateur cardiaque implantable suivant l'une des revendications 1 et 3 à 6, caractérisé par le fait que la structure possède des facettes.

8. Stimulateur cardiaque implantable suivant l'une des revendications 1 a 7, caractérisé par le fait que la paroi intérieure de la cavité est formée, au niveau de zones (21,22) différentes, par des matériaux possédant des duretés différentes.

9. Stimulateur cardiaque implantable suivant l'une des revendications 1 à 8, caractérisé par le fait que la cavité (15) est remplie avec un milieu fluide (19) possédant une densité et une viscosité déterminées.

10. Stimulateur cardiaque implantable suivant l'une des revendications 1 à 9, caractérisé par le fait que le corps est réalisé en un matériau rigide.

11. Stimulateur cardiaque implantable suivant les revendications 1 à 9, caractérisé par le fait que le corps (29,54) est constitué de plusieurs matériaux.

12. Stimulateur cardiaque implantable suivant la revendication 10, caractérisé par le fait que le matériau présente une aimantation permanente.

**13.** Stimulateur cardiaque implantable suivant l'une des revendications 1 à 12, caractérisé par le fait que le corps (4,54) possède une structure superficielle.

**14.** Stimulateur cardiaque implantable suivant l'une des revendications 1 à 12, caractérisé par le fait que le corps (54) possède une surface lisse.

**15.** Stimulateur cardiaque implantable suivant l'une des revendications 13 à 14, caractérisé par le fait que la surface du corps est formée par un matériau formant couche extérieure.

**16.** Stimulateur cardiaque implantable suivant l'une des revendications 1,3,6 à 11, 13 à 15, caractérisé par le fait que le corps (4) et/ou le corps creux est (sont) constitué(s) par un polyèdre régulier.

**17.** Stimulateur cardiaque implantable suivant la revendication 1, caractérisé par le fait que le corps creux est rempli par plusieurs corps possédant la forme de particules.

**18.** Stimulateur cardiaque implantable suivant la revendication 17, caractérisé par le fait que les particules (30) sont conductrices et que des parties (31,32) de la paroi intérieure du corps creux sont conductrices et sont raccordées à une source de tension.

**19.** Stimulateur cardiaque implantable suivant la revendication 1, caractérisé par le fait qu'un microphone (5) est prévu en tant que transducteur.

**20.** Stimulateur cardiaque implantable suivant la revendication 1, caractérisé par le fait que le signal électrique commande la fréquence du générateur d'impulsions après une amplification et/ou un traitement du signal.

FIG 1

FIG 2

FIG 3

Signal

Zeit

FIG 4

FIG 5

FIG 6

FIG 7

FIG 8

# FIG 9

FIG 10

30

31

32

y

z

x

FIG 11

33

30

31

32

y

x

FIG 12

31

30

33

32

x

y

# FIG 13

# FIG 14

# FIG 15

# FIG 16

# FIG 17

# FIG 18

Signal

Zeit